# FASCICULE DE BREVET EUROPEEN

(11) **EP 1 645 232 B1**
(45) Date de publication et mention de la délivrance du brevet: **31.12.2008**
(21) Numéro de dépôt: 05356178.3
(22) Date de dépôt: 28.09.2005
(51) Int. Cl.: A61B 17/04, A61B 17/068

(54) **Appareil de stockage, distribution et pose d'attaches chirurgicales**
Gerät zum Speichern, Zuführen und Einbringen chirurgischer Klammern
Device for stocking, delivering and deploying surgical staples

(30) Priorité: 06.10.2004 FR 0410585
(43) Date de publication de la demande: 12.04.2006
(73) Titulaire: SOFRADIM PRODUCTION, 01600 Trévoux (FR)
(72) Inventeur: Bailly, Pierre, 69300 Caluire (FR); Therin, Michel, 69004 Lyon (FR)
(74) Mandataire: Brédeville, Odile Marie

(56) Documents cités:
- WO-A-00/49950
- WO-A-03/043503
- WO-A-03/075773
- FR-A- 2 308 349
- US-A- 5 376 097
- US-A- 5 601 571
- US-A1- 2002 087 170

## Description

La présente invention concerne un appareil de distribution et de pose d'une attache chirurgicale en « I » couché, par exemple pour fixer des renforts pariétaux et viscéraux, ledit appareil et ladite attache permettant une pose non traumatisante pour les tissus.

On connaît déjà de WO03/075773 un appareil de distribution et de pose d'attaches chirurgicales en « I ». Les attaches décrites dans ce document comprennent une barre d'ancrage, une barre d'arrêt et une barrette de liaison. Elles sont introduites dans les tissus à fixer par le moyen d'une aiguille creuse fendue, en biseau et à bout acéré qui traverse lesdits tissus pour délivrer l'attache à l'endroit où elle doit prendre sa fonction. Ainsi, dans ce document, l'aiguille traverse les chairs et, du fait de sa forme creuse, peut provoquer des saignements ainsi qu'un phénomène de « carottage ». Par « carottage », on entend un prélèvement de chair correspondant au volume intérieur de la partie de l'aiguille creuse ayant pénétré dans les chairs. Ainsi, l'appareil décrit dans ce document présente des risques d'hémorragies.

Par ailleurs, dans WO03/075773, lorsque l'aiguille à pointe en biseau pique le tissu de la prothèse au niveau d'un croisement de fil ou au niveau d'un fil, elle traverse ce ou ces fils, en séparant éventuellement les brins composant les fils, ce qui peut provoquer résistance et bourrage. Cela endommage les fils et donc la prothèse. La résistance provoquée peut également bloquer le mécanisme de l'appareil et le rendre inutilisable.

Un autre problème rencontré avec l'appareil décrit dans WO03/075773 est que, du fait de la nécessaire pénétration de l'aiguille en biseau dans les chairs, l'appareil ne permet pas d'agrafer une prothèse au niveau d'une structure anatomique de faible épaisseur, c'est-à-dire en particulier d'une épaisseur inférieure à la longueur de la partie d'aiguille traversant les chairs.

La présente invention vise à remédier à ces problèmes en proposant un appareil de distribution d'une attache particulière en « I » couché, apte à délivrer ladite attache de façon non traumatisante pour les chairs et sans provoquer de phénomène de carottage et en limitant les risques de saignement.

La présente invention porte sur un appareil de stockage, de distribution et de pose d'attaches chirurgicales (A) en « I » couché comprenant un corps de poignée (B) équipé d'au moins un moyen de commande, et un élément longiforme (C), rapporté et fixé sur la poignée, appareil dans lequel l'élément longiforme (C) est composé d'un corps de forme générale tubulaire contenant :
- à proximité de son extrémité distale, un coulisseau-magasin contenant lui-même des attaches en « I » couché ayant une barre d'ancrage, une barre d'arrêt et une barrette de liaison, ledit coulisseau-magasin étant déplaçable dans l'élément longiforme par une tige de commande actionnable par le moyen de commande de la poignée et, d'autre part solidaire d'un canon d'éjection creux, fendu et longitudinal, saillant de l'extrémité distale dudit coulisseau-magasin et pouvant saillir de l'élément longiforme (C),
- dans son corps tubulaire, d'une part, des moyens de distribution une à une des attaches (A) stockées, cette distribution comprenant le transfert de la barre d'ancrage de la première attache dans un logement d'accueil disposé dans le prolongement du canon d'éjection, et d'autre part, un poussoir d'éjection, déplaçable par le moyen de commande de la poignée et disposé dans l'alignement du canon d'éjection pour pousser dans celui-ci la barre d'ancrage de la première attache,
caractérisé en ce que :
- la partie distale de la barre d'ancrage des attaches (A) est de forme conique et l'extrémité distale de cette partie distale est de forme hémisphérique,
- la surface de l'extrémité distale du canon d'éjection est disposée selon un plan substantiellement perpendiculaire à l'axe longitudinal du corps tubulaire.

La présente invention porte encore sur une attache chirurgicale en « I » couché comprenant une barre d'ancrage, une barre d'arrêt et une barrette de liaison, caractérisée en ce que la partie distale de la barre d'ancrage de l'attache est de forme conique et l'extrémité distale de cette partie distale est de forme hémisphérique.

La présente demande porte encore sur un ensemble comprenant au moins une attache chirurgicale (A) en « I » couché et un appareil de stockage, de distribution et de pose de telles attaches (A), comprenant un corps de poignée (B) équipé d'au moins un moyen de commande, et un élément longiforme (C), rapporté et fixé sur la poignée, appareil dans lequel l'élément longiforme (C) est composé d'un corps de forme générale tubulaire contenant :
- à proximité de son extrémité distale, un coulisseau-magasin contenant lui-même les attaches (A) en « I » couché ayant une barre d'ancrage, une barre d'arrêt et une barrette de liaison, ledit coulisseau-magasin étant déplaçable dans l'élément longiforme par une tige de commande actionnable par le moyen de commande de la poignée et, d'autre part solidaire d'un canon d'éjection creux, fendu et longitudinal, saillant de l'extrémité distale dudit coulisseau-magasin et pouvant saillir de l'élément longiforme (C),
- dans son corps tubulaire, d'une part, des moyens de distribution une à une des attaches (A) stockées, cette distribution comprenant le transfert de la barre d'ancrage de la première attache dans un logement d'accueil disposé dans le prolongement du canon d'éjection, et d'autre part, un poussoir d'éjection, déplaçable par le moyen de commande de la poignée et disposé dans l'alignement du canon d'éjection pour pousser dans celui-ci la barre d'ancrage de la première attache,
caractérisé en ce que :
- la partie distale de la barre d'ancrage des attaches (A) est de forme conique et l'extrémité distale de cette partie distale est de forme hémisphérique,
- la surface de l'extrémité distale du canon d'éjection est disposée selon un plan substantiellement perpendiculaire à l'axe longitudinal du corps tubulaire.

Dans la présente demande, on entend par extrémité distale d'une pièce l'extrémité la plus éloignée de l'utilisateur de l'appareil et par extrémité proximale, l'extrémité la plus proche de l'utilisateur de l'appareil.

La pose d'une attache avec l'appareil selon l'invention est non traumatisante pour les chairs. En effet, le canon d'éjection de l'appareil de l'invention présente une extrémité distale plate qui n'incise ni les chairs ni la prothèse, ni ne les pénètre.

Ainsi, dans une forme de réalisation de l'invention, le canon d'éjection peut être en métal et rapporté sur le coulisseau-magasin.

Dans une forme préférée de réalisation de l'invention, le canon d'éjection est en matière plastique et est moulé de corps avec le coulisseau-magasin. De préférence, la surface de l'extrémité distale du canon d'éjection est émoussée. Une telle configuration est préférée car un tel canon d'éjection est particulièrement non traumatisant pour les chairs. Un tel canon d'éjection ne provoque donc pas de phénomène de carottage. II ne provoque donc pas de prélèvement de chair susceptible de déclencher des hémorragies.

Par ailleurs, la forme, en portion de cône terminée par une demi-sphère, et la nature spécifiques de l'attache selon l'invention permettent à cette dernière de se frayer un passage tout d'abord à travers les mailles de la prothèse sans en endommager les fils et donc sans abîmer cette prothèse, puis ensuite à travers les tissus humains sans provoquer de saignement. Les risques de bourrage et d'hémorragies sont également évités.

Dans une forme préférée de réalisation de l'invention, l'attache A est en matière plastique biocompatible. De préférence, elle est réalisée en matière plastique biorésorbable. De préférence encore, la matière plastique biorésorbable est un polymère d'acide lactique.

Grâce à sa nature plastique, l'attache glisse et écarte les fils de la prothèse sans les endommager pour se ménager un passage au centre de la maille de la prothèse.

Enfin, il est possible, grâce à l'ensemble selon l'invention d'agrafer une prothèse au niveau d'une structure anatomique de faible épaisseur, par exemple au niveau du ligament de Cooper, dont l'épaisseur peut être inférieure à 3 mm, et ce sans que la proximité d'une structure solide, comme l'os pubien, ne crée un obstacle. En effet, dans le cas où l'attache selon l'invention entre en contact avec l'os pubien, son extrémité hémisphérique lui permet de glisser en douceur sur cet os et de pivoter aisément dans les tissus pour se mettre en place et assurer son rôle de fixation efficace.

En effet, avec l'appareil selon l'invention, du fait que le canon d'éjection ne pénètre ni la prothèse, ni les tissus humains, l'attache dispose d'un espace plus important dans les tissus humains pour effectuer la rotation de la barre d'ancrage lors du positionnement de l'attache au moment de son éjection, par rapport au cas d'un appareil de l'art antérieur pour lequel une aiguille en biseau pénètre également dans les tissus. En particulier, selon la présente invention, le canon d'éjection ne présentant pas d'embout en biseau, l'attache peut s'échapper du canon d'éjection plus rapidement.

Dans une forme préférée de réalisation de l'invention, la longueur de la partie saillante du canon d'éjection au moment de l'éjection est inférieure ou égale à 3 mm.

Dans une forme préférée de réalisation de l'invention, la longueur totale L' de l'attache (A) dans sa position de fonctionnement est inférieure ou égale à 10,5 mm.

Dans une forme préférée de réalisation de l'invention, la hauteur totale H de l'attache (A) dans sa position de fonctionnement est inférieure ou égale à 6,1 mm.

En particulier, selon l'invention, l'encombrement de l'attache est minimal. Il est ainsi possible de stocker par exemple jusqu'à vingt attaches au sein du coulisseau-magasin.

Dans une forme préférée de réalisation de l'invention, la poignée est munie d'un double système anti-retour assurant une utilisation adéquate de l'appareil et évitant son enrayage.

Dans une forme préférée de réalisation de l'invention, l'élément longiforme C comprend un système de pas-à-pas apte à déplacer les attaches A stockées d'un pas P de valeur égale à la longueur L de la barre d'arrêt d'une attache A, ce système de pas-à-pas comprenant une crémaillère effectuant un mouvement de tiroir à chaque éjection et étant munie d'une navette montée coulissante sur cette crémaillère, cette navette coopérant avec des crans fixes du coulisseau-magasin pour progresser d'un pas P sur la crémaillère à chaque éjection.

D'autres caractéristiques et avantages ressortiront de la description qui suit en référence au dessin schématique annexé représentant une forme d'exécution de l'ensemble selon l'invention.
La figure 1 est une vue de côté d'un appareil selon l'invention au repos ;
La figure 2 est une vue de côté d'une attache selon l'invention ;
La figure 3 est une vue en coupe de l'élément longiforme,
La figure 4 est une vue partielle en coupe montrant le système de pas-à-pas et la navette,
Les figures 5 à 7 sont des vues partielles en coupe montrant le fonctionnement du système de pas-à-pas,
La figure 8 est une vue en coupe de la poignée en position de repos,
Les figures 9 à 11 montrent le fonctionnement du double système anti-retour de la poignée,
Les figures 12 à 17 montrent les différentes phases de l'éjection d'une attache avec l'appareil selon l'invention,
La figure 18 est une vue en perspective éclatée à échelle agrandie de l'extrémité antérieure de l'élément longiforme et du coulisseau-magasin,
La figure 19 est une vue en coupe de l'extrémité antérieure de l'élément longiforme.

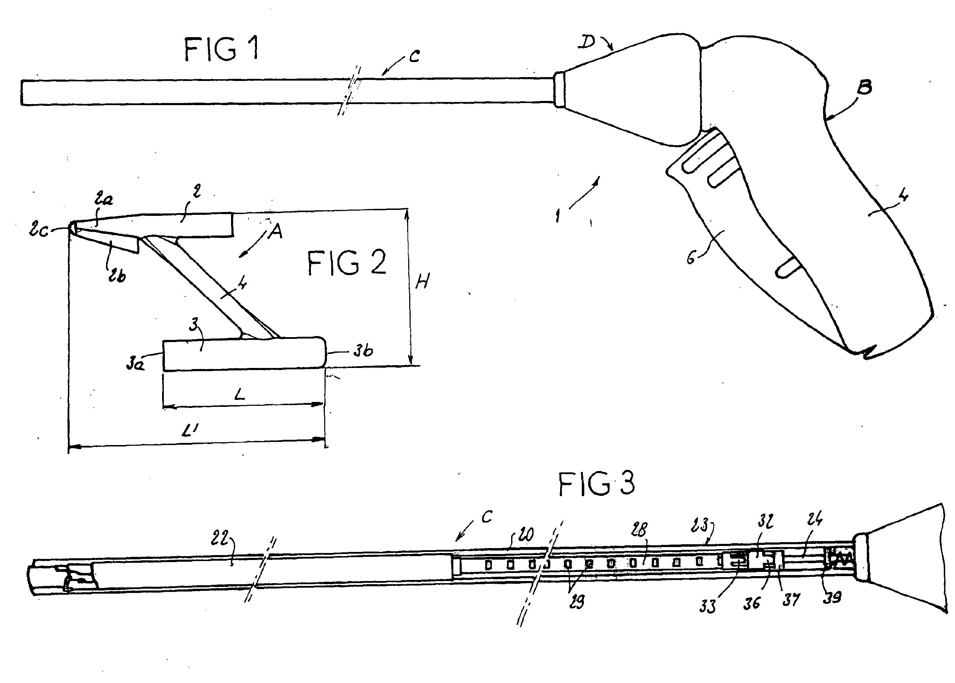

L'appareil 1 représenté à la figure 1 est destiné à distribuer des agrafes A ayant, comme montré à la figure 2, la forme générale d'un « I » couché et composé de deux barres parallèles, respectivement, d'ancrage 2 et d'arrêt 3, reliées par une barrette de liaison inclinée 4. La partie distale 2a de la barre d'ancrage 2 est de forme conique. Cette partie distale 2a peut comprendre un cran 2b en saillie pour protéger la jonction barre d'ancrage-barrette de liaison qui est sollicitée au moment de l'éjection et de la mise en place de l'agrafe. L'extrémité distale 2c de la barre d'ancrage 2 est de forme hémisphérique. La partie distale de la barre d'ancrage 2 est engagée en premier dans la prothèse et dans le corps humain. Sa forme particulière décrite ci-dessus permet un engagement facilité de l'attache à travers la prothèse et les tissus humains. La partie hémisphérique combinée à la partie conique permet à l'attache de se ménager un passage à travers les mailles de la prothèse en glissant le long des fils et en les écartant sans les endommager. Ainsi, la partie distale de la barre d'ancrage 2 passe à travers une maille de la prothèse sans provoquer de résistance ou de bourrage et pénètre ensuite facilement dans les tissus humains grâce à sa forme conique et effilée, sans toutefois traumatiser ces derniers du fait de la forme hémisphérique, donc douce et émoussée, de son extrémité distale.

L'attache A est réalisée en matière plastique, notamment en matériau biocompatible, de préférence biorésorbable.

La barrette de liaison 4 est reliée environ au centre de la barre d'ancrage 2. La barre d'arrêt 3 comprend une partie distale 3a et une partie proximale 3b. La barrette de liaison 4 est reliée à la barre d'arrêt 3 à un point proche du centre de cette barre d'arrêt 3, légèrement décalé vers sa partie proximale 3b. La barrette de liaison est inclinée d'environ 45° par rapport aux barres d'ancrage et d'arrêt. Cette forme particulière de réalisation de l'attache permet de réduire l'encombrement de l'attache tout en lui conférant la solidité suffisante pour assurer une fixation efficace de la prothèse dans les tissus : il est ainsi possible à la fois d'utiliser cette attache pour agrafer des structures anatomiques de faible épaisseur et de stocker un grand nombre d'attaches au sein de l'appareil 1 comme il sera vu plus loin. La longueur totale L' de l'attache (A), dans sa position de fonctionnement telle que représentée à la figure 2, à savoir de l'extrémité distale 2c de sa barre d'ancrage 2 à l'extrémité proximale 3b de sa barre d'arrêt 3, est de préférence inférieure ou égale à 10,5 mm. La hauteur H de l'attache (A), dans sa position de fonctionnement telle que représentée à la figure 2 est de préférence inférieure ou égale à 6,1 mm.

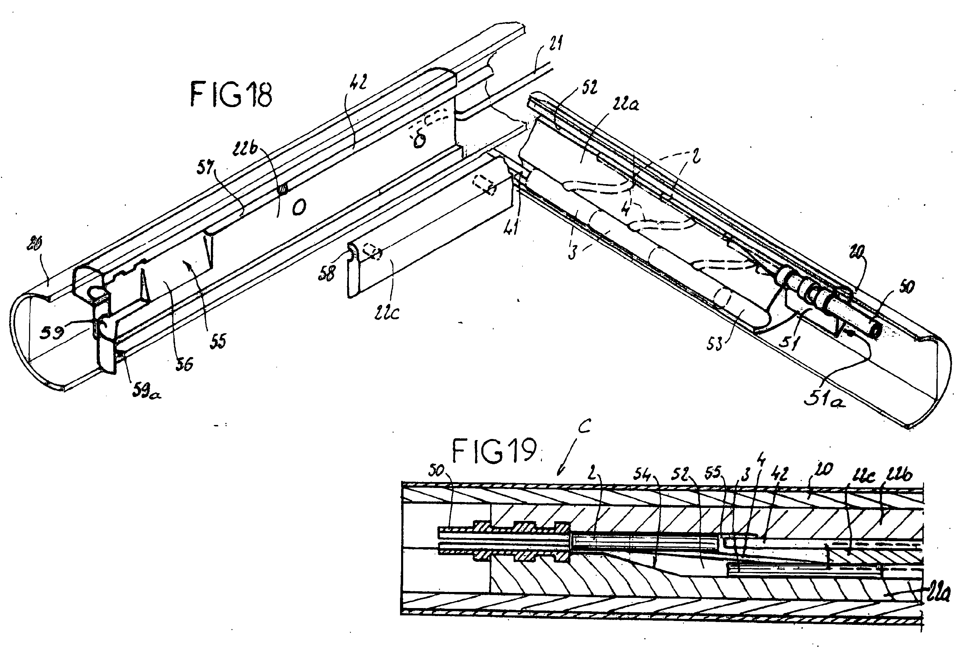

Comme montré à la figure 1, l'appareil 1 est composé de deux éléments, à savoir un ensemble de préhension B et un élément longiforme C reliés l'un à l'autre à l'aide d'une douille D. Le canon d'éjection 50 (voir figure 18) étant excentré, la douille D permet de faire pivoter l'élément longiforme C et de le maintenir fixe en rotation dans la position préférée du chirurgien pour faire saillir le canon d'éjection 50.

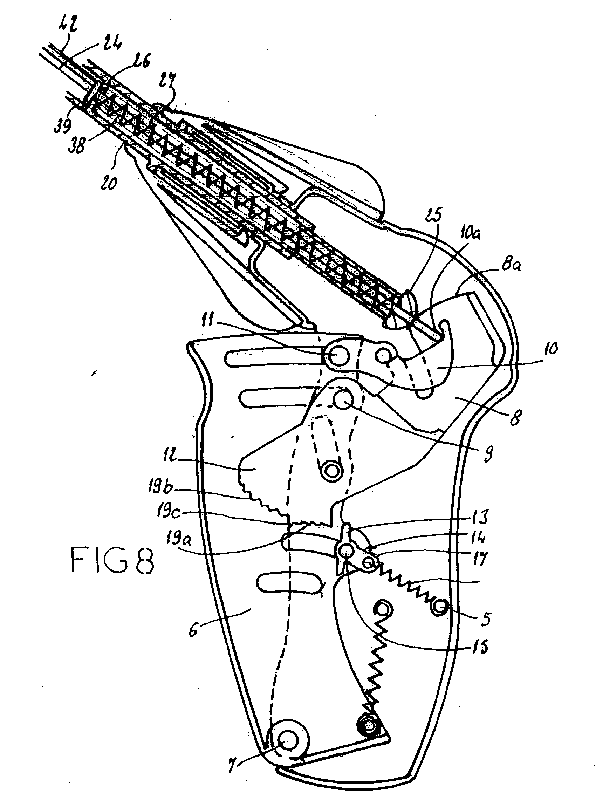

Comme montré à la figure 8, l'élément de préhension B comprend un corps de poignée 4 réalisé en deux parties symétriques assemblées par soudage à ultrasons ou par des rivets ou vis 5, une poignée 6 articulée sur un axe transversal 7 du corps, un premier levier de commande 8 articulé sur un axe transversal 9 du corps et présentant un profil en came 8a, et un deuxième levier de commande 10 articulé sur un axe transversal 11 du corps et présentant un profil en came 10a.

La partie inférieure du premier levier de commande 8 forme un arc de cercle 12 muni de dents destinées à coopérer avec la branche 13 d'une molette 14 articulée sur un axe transversal 15 du corps tout au long du geste chirurgical, à savoir une phase de positionnement de l'appareil au cours de laquelle le chirurgien peut faire sortir le canon d'éjection 50 (voir figure 18) sans procéder à l'éjection elle-même, puis une phase d'éjection et enfin une phase de retour au repos de l'appareil, comme cela sera expliqué plus loin. La molette 14 comprend une deuxième branche 17, de retenue, reliée à un ressort 18 dont l'extrémité fixe est attachée au corps de poignée 4. L'arc de cercle 12 comprend une première série de dents 19a à pentes faibles et une deuxième série de dents 19b à pentes fortes, les deux séries de dents étant séparées par une dent 19c de taille supérieure.

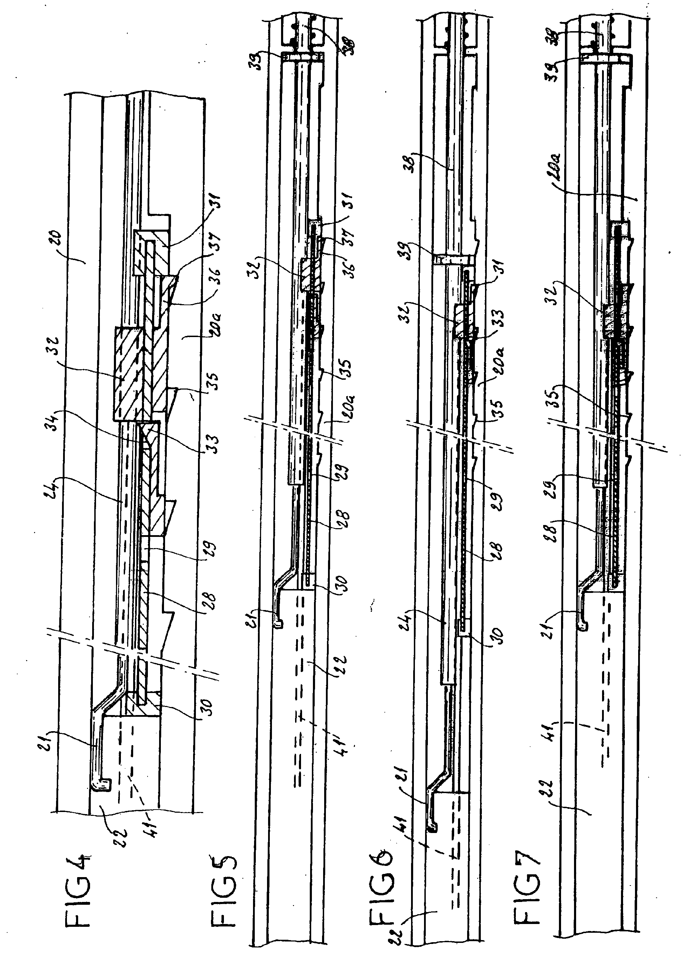

Comme montré figure 3, l'élément longiforme C comprend un corps tubulaire 20 dans lequel est monté coulissant un coulisseau-magasin 22. Le corps 20 comprend également un système de pas-à-pas 23 dont le fonctionnement est expliqué plus en détails aux figures 4 à 7.

Comme montré à la figure 4, le coulisseau-magasin 22 est relié à une tige de commande 24 à l'aide d'une tige axiale 21 de plus petit diamètre coudée et contrecoudée. Comme montré à la figure 8, la tige de commande 24 prend appui sur le profil en came 10a du deuxième levier de commande 10. Le corps tubulaire 20 est lui-même composé de deux coquilles semi cylindriques en matière plastique assemblées dans leur plan diamétral, par soudure, emboîtage ou collage, chacune des coquilles étant elle-même monolithique. De préférence, le corps 20 est disposé dans une frette tubulaire métallique non représentée servant à rigidifier l'élément longiforme C.

Comme montré figure 8, le corps tubulaire 20 comprend également un piston 25 pénétrant dans le corps de poignée 4 et maintenu en appui sur le profil en came 8a du premier levier de commande 8 au moyen d'un ressort 27 prenant appui sur une collerette 26 du corps tubulaire 20. Le piston 25 est solidaire d'un tube 38 qui le traverse et qui traverse également le ressort 27. L'extrémité distale de ce tube 38 est munie d'une collerette 39 sur laquelle est fixée une tige poussoir 42 s'étendant dans le coulisseau-magasin.

Le système de pas-à-pas 23 est décrit sur la figure 3 et sur la figure 4 qui est une vue en coupe par un plan décalé de 90° par rapport au plan de coupe de la figure 3. Il comprend une crémaillère 28, par exemple en métal, munie d'encoches 29 espacées régulièrement selon un pas P de valeur égale à la longueur L (figure 2) de la barre d'arrêt 3 d'une attache A. La crémaillère 28 est munie à ses extrémités d'un patin distal 30 et d'un patin proximal 31, de préférence en matière plastique. Sur la crémaillère 28, est montée coulissante une navette 32, de préférence en matière plastique. Cette navette 32 comporte une patte distale 33 présentant une face inclinée 34 autorisant le déplacement relatif, dans le sens proximal, de la crémaillère 28 par rapport à la navette 32. Cette même patte 33 s'encliquète dans les encoches 29 de la crémaillère 28 pour empêcher le déplacement relatif dans le sens distal, de la crémaillère 28 par rapport à la navette 32, comme montré à la figure 4.

Le système de pas-à-pas 23 comprend également une série de crans 35 ménagés sur la face interne d'une coquille 20a du corps tubulaire 20 en regard de la crémaillère 28 et espacés régulièrement selon un pas P de valeur égale à la longueur L (figure 2) de la barre d'arrêt 3 d'une attache A. La navette 32 comporte une patte proximale 36 présentant une saillie 37 autorisant le déplacement relatif, dans le sens distal, de la navette et de la crémaillère, par rapport à la coquille 20a. Cette saillie 37 s'encliquète dans les crans 35 de la coquille 20a pour empêcher le retour de la navette 32 dans le sens proximal.

Sur la navette 32 est montée une tige de butée 41 qui s'étend longitudinalement et pénètre dans le coulisseau-magasin 22 dans la rainure 53 (voir figure 18) servant de logements aux barres d'arrêt 3 des attaches A.

Comme montré à la figure 18, le coulisseau-magasin est formé par deux coquilles en matière plastique 22a et 22b s'assemblant par collage ou soudure, selon leur plan médian longitudinal et vertical, en enserrant une plaquette intercalaire 22c.

La coquille 22a est solidaire, à son extrémité distale, d'un canon d'éjection 50. Ce canon d'éjection 50 est creux et fendu et la surface de son extrémité distale est disposée selon un plan substantiellement perpendiculaire à l'axe longitudinal du corps tubulaire 20. Ce canon d'éjection 50 est destiné à recevoir la barre d'ancrage 2 d'une attache A. Ce canon d'éjection 50 est en matière plastique et est moulé de corps avec le coulisseau-magasin 22.

La coquille 22a comporte, près de ses bords, deux rainures longitudinales, une rainure 52, servant de logements aux barres d'ancrage 2 des attaches A, et une rainure 53 servant de logements aux barres d'arrêt 3 des mêmes attaches. Ces deux rainures sont dans un même plan vertical, distinct de celui passant par le canon d'éjection 50. La rainure 53 servant de logements aux barres d'arrêt 3 débouche librement de l'extrémité antérieure de la coquille 22a, tandis que la rainure 52 servant de logements aux barres d'ancrage 2 vient mourir sur un plan incliné visible figure 19. Ce dernier favorise le déplacement transversal de la barre d'ancrage 2 de la première attache jusqu'à ce que cette barre vienne derrière et dans l'axe longitudinal du canon d'éjection, dans un logement d'accueil 55 comme montré à la figure 18.

La coquille 22a comporte encore, à côté du canon d'éjection 50, une nervure 51 saillant vers le bas et présentant, au-dessus de la trajectoire de sortie d'une barre d'arrêt de la rainure 53 servant de logements aux barres d'arrêt 3, une face pentue 51 a.

La figure 19, qui représente l'appareil en position de repos, montre que, dans cette position, la tige poussoir 42 est en retrait de l'extrémité postérieure du canon d'éjection 50 pour dégager le logement d'accueil 55 ménagé dans l'autre coquille 22b.

La figure 18 montre que le logement d'accueil 55 pour la barre d'ancrage 2 de la première attache est prolongé vers le bas par une face inclinée 56 de soutien de la barrette de liaison 4 de l'attache. Cette coquille 22b comporte également une rainure semi cylindrique 57 qui, avec une rainure complémentaire 58 ménagée dans la plaquette 22c, forme un canal de guidage pour la tige poussoir 42. Ce canal est coaxial au canon d'éjection 50. L'extrémité proximale de la coquille 22b est liée à la tige axiale 21 elle-même reliée à la tige de commande 24. L'extrémité distale de la coquille 22b est munie, dans sa partie venant sous le canon d'éjection 50, d'un doigt transversal 59 saillant en direction de l'autre coquille, présentant une face pentue 59a et dont l'utilité sera précisée ultérieurement.

La plaquette intercalaire 22c a une largeur lui permettant de s'insérer entre les rainures 52 et 53 de la coquille 22a et une épaisseur lui permettant de former, avec cette coquille 22a, un couloir permettant de libérer le passage pour les barrettes de liaison 4 des attaches A.

Le coulisseau-magasin 22 ainsi formé a une longueur lui permettant de stocker par exemple vingt attaches A.

Lorsque l'appareil est au repos, la poignée 6 est dans la position représentée à la figure 8, avec la tige de commande 24 en appui sur le profil en came 10a du deuxième levier de commande 10 et le piston 25 en appui sur le profil en came 8a du premier levier de commande. Le coulisseau-magasin 22 est en position rétractée dans le corps tubulaire 20. Le canon d'éjection 50 est rétracté dans le corps et la tige poussoir 42 qui dégage le logement d'accueil 55 n'exerce aucun effort sur la barre d'ancrage 2 disposée dans ce logement. De même, les barres d'arrêt 3 des attaches A en attente dans le magasin sont en contact les unes avec les autres, tandis que la barre d'arrêt de la dernière attache est à proximité, avec ou sans contact, de l'extrémité de la tige de butée 41.

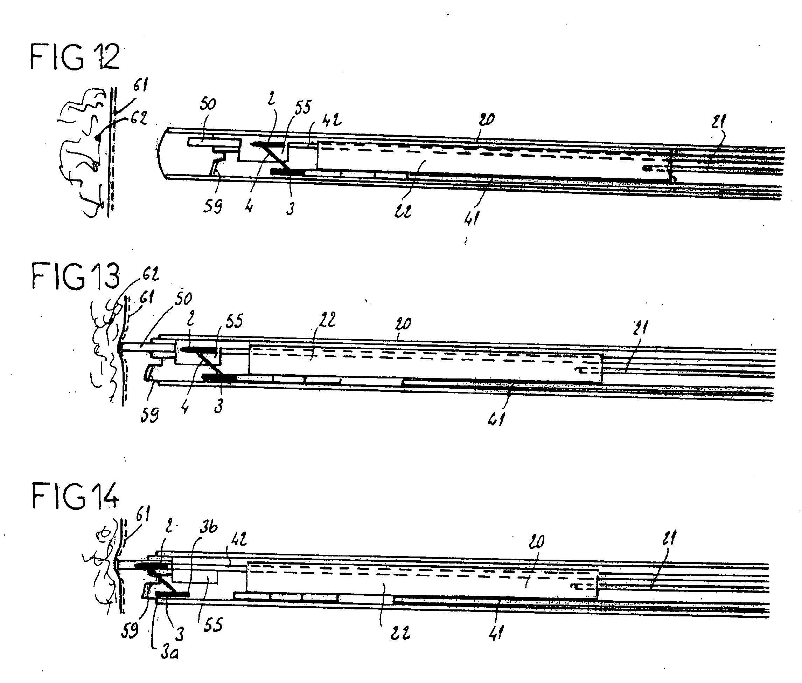

En utilisation, par exemple par voie laparoscopique, l'élément longiforme C de l'appareil est introduit, avec le canon d'éjection 50 rentré, dans un trocart. L'engagement est effectué jusqu'à proximité, voire contact avec la prothèse 61 qu'il faut agrafer aux tissus humains 62, comme montré sur la figure 12. Le chirurgien a alors la possibilité de faire saillir le canon d'éjection 50 sans pour cela déclencher l'éjection de l'attache A, comme montré sur la figure 13. Pour ce faire, le chirurgien presse la poignée 6. Sur une première partie de la course, le deuxième levier de commande 10 pivote autour de son axe 11. Le profil en came 10a appuie sur la tige 24 qui se déplace dans le sens distal et pousse le coulisseau-magasin 22 également dans le sens distal en faisant saillir le canon d'éjection 50, hors du corps tubulaire 20.

Durant cette première partie de course également, la branche 13 de la molette 14 coopère avec la série de dents 19a de l'arc de cercle 12 du premier levier de commande 8. Tant que la branche 13 n'a pas dépassé la dent de taille supérieure 19c, le chirurgien peut relâcher son effort sur la poignée et revenir à la position de repos. En effet, l'extrémité de la branche 13 étant arrondie, elle ne s'engrène pas de façon définitive dans les pentes faibles des dents 19a qui autorisent ainsi la branche 13 à retourner à sa position initiale. Ainsi, tant qu'il n'exerce pas un effort suffisant pour dépasser la dent 19a, le chirurgien peut faire saillir le canon d'éjection 50 puis le faire à nouveau rentrer dans le corps tubulaire 20 sans déclencher l'éjection de l'attache A. Cela lui permet de tester divers positionnements de l'appareil pour le placer finalement idéalement par rapport aux tissus (61, 62) qu'il doit agrafer.

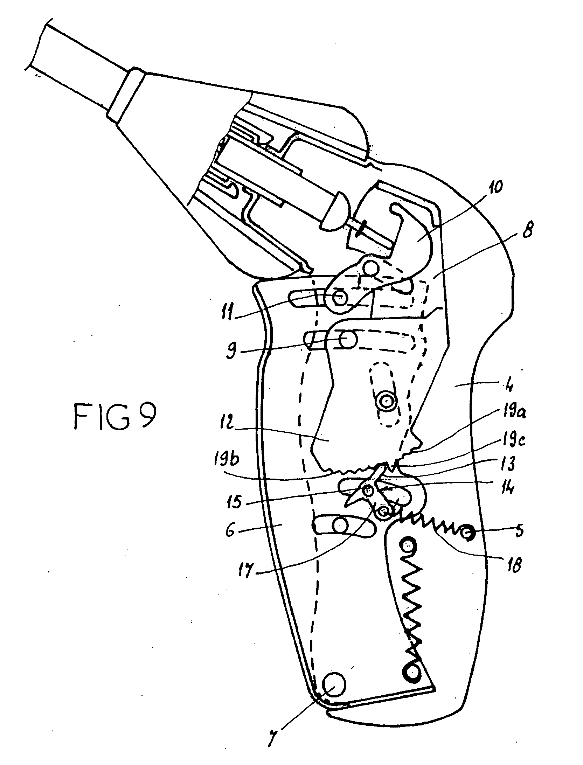

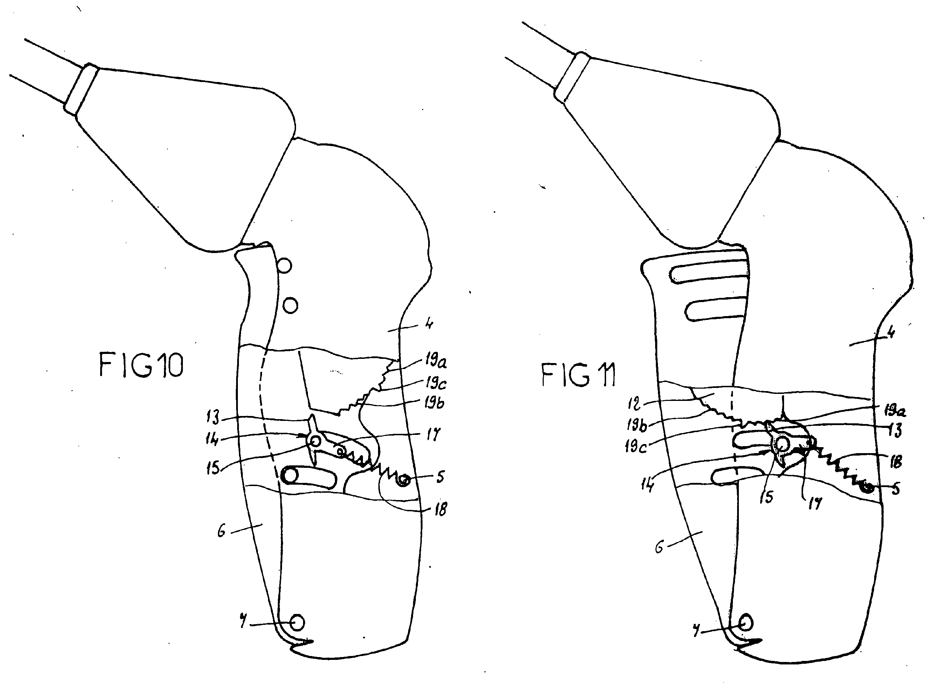

Une fois que le chirurgien a suffisamment pesé sur la poignée 6 pour que la branche 13 de la molette 14 dépasse la dent 19c, le retour en arrière n'est plus possible comme montré sur la figure 9. Sur cette figure, la branche 13 de la molette 14 est bloquée contre la dent de taille supérieure 19c. Durant la deuxième partie de course, le chirurgien doit procéder à l'éjection de l'attache. Pour éviter une utilisation mal adaptée de l'appareil, qui pourrait conduire à son enrayage, le geste du chirurgien est guidé jusqu'à sa complète réalisation. Ainsi, durant cette deuxième partie de course, la branche 13 de la molette 14 coopère avec la série de dents à pentes fortes 19b pour permettre la progression du geste, dent par dent, tout en empêchant le relâchement de la poignée et donc le retour en arrière. Ainsi, le chirurgien est obligé d'appuyer sur la poignée jusqu'à la fin de sa course, comme montré à la figure 10 pour libérer la branche 13 des dents 19b et ainsi déclencher l'éjection de l'attache A.

Durant cette deuxième partie de course, le deuxième levier de commande 10 a provoqué le déplacement du piston 25, donc du tube 38 et de la tige poussoir 42, le déplacement de la tige poussoir 42 s'effectuant par rapport au coulisseau-magasin 22 qui reste temporairement fixe.

La tige poussoir 42 est donc déplacée longitudinalement afin que son extrémité vienne en contact avec l'extrémité proximale de la barre d'ancrage 2 de l'attache A disposée dans le logement d'accueil 55, et pousse cette barre d'ancrage dans le canon d'éjection 50, comme montré à la figure 14.

Le déplacement de la barre d'ancrage 2 tire la barrette de liaison 4 et la barre d'arrêt 3 de l'attache en cours de distribution. L'extrémité distale 3a de la barre d'arrêt 3 vient en butée contre le doigt 59, ce qui provoque le basculement vers le haut de cette barre jusqu'à la redresser parallèlement au renfort 61. Durant ce mouvement, son extrémité proximale 3b rencontre la face pentue 51a de la nervure 51 qui chasse transversalement cette extrémité, afin que, lors du redressement de la barre 3, cette extrémité ne vienne pas buter contre le canon d'éjection 50 mais au contraire s'en éloigne.

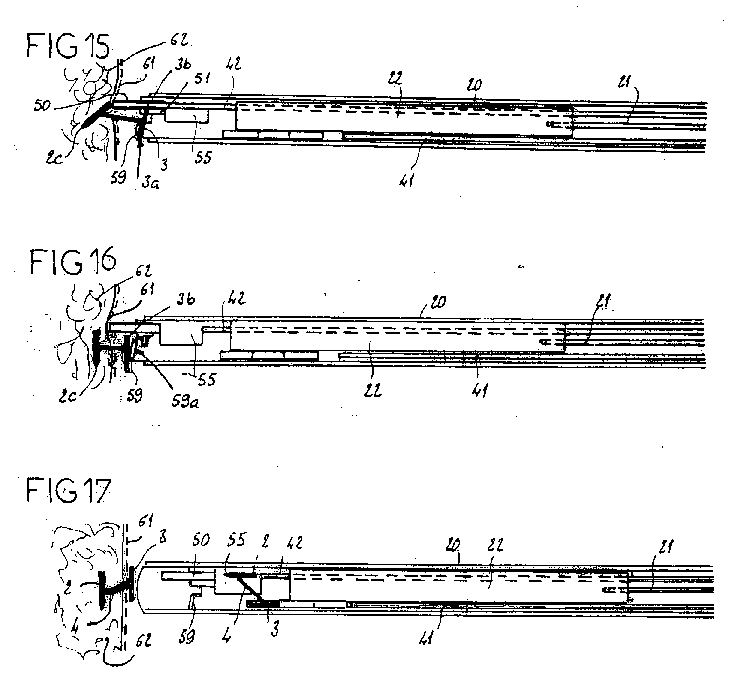

Durant ce mouvement, la barre d'ancrage 2 avance dans le canon d'éjection 50. Quand la barrette de liaison 4 échappe de celle-ci, la barre 2 peut commencer à basculer avec l'aide de la barre d'arrêt 3. En effet, par la barrette de liaison 4, le mouvement organisé de la barre d'arrêt 3 a pour conséquence de retenir la barre d'ancrage 2 qui est ainsi forcée à se redresser et à s'ancrer dans les tissus biologiques 62, comme montré à la figure 15. Du fait de la forme non biseautée du canon d'éjection 50 et du faible encombrement de la barre d'ancrage 2, cette dernière s'échappe rapidement du canon d'éjection 50 et n'a ensuite besoin que de peu d'espace pour effectuer son redressement et sa rotation comme montré sur les figures 15 et 16. L'appareil 1 selon l'invention et son attache permettent ainsi d'agrafer des prothèses au niveau de structures anatomiques de faible épaisseur.

Enfin, pendant ou en fin de redressement de la barre d'ancrage 2, la barre d'arrêt 3 qui est sensiblement parallèle au renfort textile 61, glisse, par son extrémité distale 3a, sur la face pentue 59a du doigt 59 qui la chasse transversalement et la libère.

La longueur de la partie saillante du canon d'éjection 50 au moment de l'éjection peut par exemple être de 3 mm.

A la fin de ce mouvement, et comme montré à la figure 16, le relâchement de l'action sur la poignée 6 permet au ressort 27 de rappeler le coulisseau-magasin 22 et la tige poussoir 42. Durant le relâchement de la poignée 6, la branche 13 de la molette 14 coopère avec les séries de dents 19a, 19b, 19c pour empêcher l'utilisateur d'appuyer de nouveau sur la poignée avant que cette dernière ne soit revenue en position de repos, comme montré sur la figure 11. Ce deuxième système anti-retour permet d'éviter là aussi une utilisation mal adaptée de l'appareil et donc son enrayage. L'arc de cercle 12 du premier levier de commande 8, les séries de dents 19a, 19b et 19c forment, avec la branche 13 de la molette 14 un double système anti-retour assurant une utilisation adéquate de l'appareil et évitant son enrayage.

Une fois la poignée 6 revenue en position de repos, l'appareil 1 peut être utilisé pour procéder aux fixations suivantes ou être extrait du corps humain, comme montré figure 17.

Pendant la distribution de l'attache A, les moyens de distribution mis en oeuvre permettent à la barre d'ancrage 2 et à la barre d'arrêt 3 de se redresser pour assurer une parfaite retenue de ces tissus.

Pendant la phase de distribution de la première attache A, comme montré aux figures 14 à 16, le logement d'accueil 55 est obturé par la tige poussoir 42 et les attaches en attente conservent leur position grâce à un patin de freinage non représenté et solidaire du coulisseau-magasin 22. Le déplacement du coulisseau-magasin 22 vers l'avant de l'appareil emmène avec lui les attaches en stock et les éloigne de l'extrémité libre de la tige de butée 41, qui n'est mise en mouvement que sur la fin du déplacement de la tige poussoir 42.

Comme montré aux figures 5 à 7, le déplacement de la tige de butée 41 est assuré par l'avancée de la navette 32 sur la crémaillère 28.

Avant utilisation de l'appareil 1, comme montré sur les figures 4 et 5, la navette 32 est enclenchée, à la fois dans un cran 35 du corps tubulaire 20 au moyen de la saillie 37 de sa patte proximale 36, et également dans une encoche 29 de la crémaillère 28 au moyen de sa patte distale 33. Au moment de l'éjection de l'attache, le piston 25, poussé par le premier levier de commande 8, avance dans le sens distal, entraînant avec lui le tube 38 et la collerette 39 de ce tube. Comme montré à la figure 6, la collerette 39 entre en contact avec le patin proximal 31 de la crémaillère 28 et entraîne dans le sens distal cette crémaillère 28. Lors de ce déplacement, la navette 32 est maintenue solidaire de la crémaillère par sa patte distale 33 enclenchée dans l'encoche 29 de la crémaillère tandis que sa patte proximale 36 se défléchit pour permettre à la saillie 37 de passer le cran 35 du corps tubulaire 20 dans lequel elle était enclenchée et d'aller s'enclencher dans le cran 35 suivant. Ainsi, la navette 32 ainsi que la tige de butée 41 qui en est solidaire progressent dans le sens distal de la longueur d'un pas P.

Après éjection de l'attache, le coulisseau-magasin 22 est rappelé par le ressort 27 et son extrémité proximale entre en contact avec le patin distal 30 de la crémaillère 28. Comme montré à la figure 7, le coulisseau-magasin 22 entraîne ainsi avec lui dans le sens proximal la crémaillère 28. Compte tenu de la face inclinée 34 de la patte distale 33 de la navette 32, la crémaillère 28 se déplace dans le sens proximal par rapport à la navette 32 qui elle, reste immobile par rapport au corps tubulaire 20 du fait de sa saillie 37 enclenchée dans le deuxième cran 35 de ce corps tubulaire 20.

Ainsi, sur la figure 7, la navette 32 a progressé sur la crémaillère 28 d'un pas P dans le sens distal par rapport à la figure 5. Il résulte de cela que la tige de butée 41, qui est solidaire de la navette 32, a également avancé d'un pas P par rapport à la position initiale qu'elle occupait. De la sorte, lors du recul du coulisseau-magasin 22, la barre d'arrêt 3 de la dernière attache vient en contact avec l'extrémité distale de la tige de butée 41 et retient la rangée d'attaches, tandis que le coulisseau-magasin termine sa rentrée dans le corps tubulaire 20.

En fin de rentrée du coulisseau-magasin, la barre d'ancrage 2 de la première attache vient en contact avec la rampe 54, montrée figure 19, rampe qui assure son transfert dans le logement d'accueil 55.

L'appareil qui a été décrit et son attache sont particulièrement adaptés pour agrafer des prothèses telles que des renforts pariétaux sans traumatisme des chairs et sans risque d'hémorragies. Ils sont aussi particulièrement utiles pour agrafer des prothèses au niveau de structures anatomiques de faible épaisseur comme par exemple le ligament de Cooper tout en assurant une fixation très efficace, et ce de préférence seulement pendant le temps nécessaire de fixation grâce au caractère de préférence biorésorbable des attaches. Par ailleurs, l'appareil et l'attache selon l'invention peuvent être utilisés avec la même efficacité pour des interventions chirurgicales par voie laparoscopique ou au contraire par voie ouverte.

## Revendications

1. Ensemble comprenant au moins une attache chirurgicale (A) en "I" couché et un appareil (1) de stockage, de distribution et de pose de telles attaches, comprenant un corps de poignée (B) équipé d'au moins un moyen de commande (8, 10), et un élément longiforme (C), rapporté et fixé sur la poignée (4), appareil dans lequel l'élément longiforme (C) est composé d'un corps de forme générale tubulaire (20) contenant :
- à proximité de son extrémité distale, un coulisseau-magasin (22) contenant lui-même des attaches (A) en « I » couché ayant une barre d'ancrage (2), une barre d'arrêt (3) et une barrette de liaison (4), ledit coulisseau-magasin (22) étant déplaçable dans l'élément longiforme (C) par une tige de commande (24) actionnable par le moyen de commande (8, 10) de la poignée (6) et, d'autre part solidaire d'un canon d'éjection (50) creux, fendu et longitudinal, saillant de l'extrémité distale dudit coulisseau-magasin (22) et pouvant saillir de l'élément longiforme (C),
- dans son corps tubulaire (20), d'une part, des moyens de distribution (23, 41) une à une des attaches (A) stockées, cette distribution comprenant le transfert de la barre d'ancrage (2) de la première attache dans un logement d'accueil (55) disposé dans le prolongement du canon d'éjection (50), et d'autre part, un poussoir d'éjection (42), déplaçable par le moyen de commande (8, 10) de la poignée (6) et disposé dans l'alignement du canon d'éjection (50) pour pousser dans celui-ci la barre d'ancrage (2) de la première attache,
**caractérisé en ce que :**
- la partie distale (2a) de la barre d'ancrage (2) des attaches (A) est de forme conique et l'extrémité distale (2c) de cette partie distale (2a) est de forme hémisphérique,
- la surface de l'extrémité distale du canon d'éjection (50) est disposée selon un plan substantiellement perpendiculaire à l'axe longitudinal du corps tubulaire (20).

2. Ensemble selon la revendication 1, **caractérisé en ce que** le canon d'éjection (50) est en matière plastique et est moulé de corps avec le coulisseau-magasin (22).

3. Ensemble selon la revendication 1 ou 2, **caractérisé en ce que** l'attache (A) est en matière plastique biocompatible.

4. Ensemble selon la revendication précédente, **caractérisé en ce que** l'attache (A) est en matière plastique biorésorbable.

5. Ensemble selon la revendication précédente, **caractérisé en ce que** la matière plastique biorésorbable est un polymère d'acide lactique.

6. Ensemble selon l'une quelconque des revendications précédentes, **caractérisé en ce que** la longueur de la partie saillante du canon d'éjection (50) au moment de l'éjection est inférieure ou égale à 3 mm.

7. Ensemble selon l'une quelconque des revendications précédentes, **caractérisé en ce que** la longueur totale L' de l'attache (A) dans sa position de fonctionnement est inférieure ou égale à 10,5 mm.

8. Ensemble selon l'une quelconque des revendications précédentes, **caractérisé en ce que** la hauteur totale H de l'attache (A) dans sa position de fonctionnement est inférieure ou égale à 6,1 mm.

9. Ensemble selon l'une quelconque des revendications précédentes, **caractérisé en ce que** la poignée (4) est munie d'un double système anti-retour (12, 13, 14, 19a, 19b, 19c), assurant une utilisation adéquate de l'appareil et évitant son enrayage.

10. Ensemble selon l'une quelconque des revendications précédentes, **caractérisé en ce que** l'élément longiforme C comprend un système de pas-à-pas (23) apte à déplacer les attaches A stockées d'un pas P de valeur égale à la langueur L de la barre d'arrêt (3) d'une attache A, ce système de pas-à-pas (23) comprenant une crémaillère (28) effectuant un mouvement de tiroir à chaque éjection et étant munie d'une navette (32) montée coulissante sur cette crémaillère (28), cette navette (32) coopérant avec des crans (35) f xes du coulisseau-magasin (22) pour progresser d'un pas P sur la crémaillère (28) à chaque éjection.

## Claims

1. Assembly comprising at least one couched I-shaped surgical fastener (A) and an appliance (1) for storing, distributing and placing such fasteners (A), comprising a handgrip body (B) equipped with at least one control means (8, 10), and an elongate element (C) attached and fixed to the handgrip (4), in which appliance the elongate element (C) is made up of a body (20) of tubular overall shape containing:
- near its distal end, a magazine slide (22) itself containing couched I-shaped fasteners (A) having an anchoring bar (2), a catching bar (3) and a connecting strip (4), said magazine slide (22) being displaceable in the elongate element (C) by a control rod (24) which can be actuated by the control means (8, 10) of the handgrip (6) and, on the other hand, secured to a slotted and longitudinal hollow ejection barrel (50) projecting from the distal end of said magazine slide (22) and able to project from the elongate element (C),
- and, in its tubular body (20), on the one hand, means (23, 41) for distributing the stored fasteners (A) one by one, this distribution involving the transfer of the anchoring bar (2) of the first fastener into a receiving seat (55) arranged in the continuation of the ejection barrel (50) and, on the other hand, an ejection plunger (42) which can be displaced by the control means (8, 10) of the handgrip (6) and which is arranged in alignment with the ejection barrel (50) so as to push the anchoring bar (2) of the first fastener into the latter,
**characterized in that:**
- the distal part (2a) of the anchoring bar (2) of the fasteners (A) is of conical shape, and the distal end (2c) of this distal part (2a) is of hemispherical shape,
- the surface of the distal end of the ejection barrel (50) is arranged in a plane substantially perpendicular to the longitudinal axis of the tubular body (20).

2. Assembly according to Claim 1, **characterized in that** the ejection barrel (50) is made of plastic and is moulded in one piece with the magazine slide (22).

3. Assembly according to Claim 1 or 2, **characterized in that** the fastener (A) is made of biocompatible plastic.

4. Assembly according to the preceding claim, **characterized in that** the fastener (A) is made of bioabsorbable plastic.

5. Assembly according to the preceding claim, **characterized in that** the bioabsorbable plastic is a lactic acid polymer.

6. Assembly according to any one of the preceding claims, **characterized in that** the length of the projecting part of the ejection barrel (50) at the moment of ejection is less than or equal to 3 mm.

7. Assembly according to any one of the preceding claims, **characterized in that** the total length L' of the fastener (A) in its operating position is less than or equal to 10.5 mm.

8. Assembly according to any one of the preceding claims, **characterized in that** the total height H of the fastener (A) in its operating position is less than or equal to 6.1 mm.

9. Assembly according to any one of the preceding claims, **characterized in that** the handgrip (4) is provided with a double non-return system (12, 13, 14, 19a, 19b, 19c) ensuring appropriate use of the appliance and avoiding its becoming jammed.

10. Assembly according to any one of the preceding claims, **characterized in that** the elongate element C comprises a step-by-step system (23) able to move the stored fasteners A by a step P having a value equal to the length L of the catching bar (3) of a fastener A, this step-by-step system (23) comprising a rack (28) effecting a drawer movement upon each ejection and being equipped with a shuttle (32) which is mounted so as to slide on this rack (28), this shuttle (32) cooperating with fixed notches (35) of the magazine slide (22) in order to advance by a step P on the rack (28) upon each ejection.

## Patentansprüche

1. Anordnung, mit zumindest einer chirurgischen Klammer (A) in Form eines liegenden "I" und einem Apparat (1) zum Speichern, Spenden und Anbringen solcher Klammern, der einen Griffkörper (B), der mit zumindest einem Steuermittel (8, 10) ausgestattet ist, und ein längliches Element (C) aufweist, das an dem Griff (4) angefügt und festgelegt ist, wobei der Apparat, bei dem das längliche Element (C) aus einem Körper (20) von allgemein rohrförmiger Form gebildet ist, enthält:
- in Nähe seines distalen Endes ein Gleitmagazin (22), das seinerseits Klammern (A) in Form eines liegenden «I» enthält, die einen Verankerungsstab (2), einen Festlegestab (3) und einen kleinen Verbindungsstab (4) aufweisen, wobei das Gleitmagazin (22) in dem länglichen Element (C) mittels eines Steuerschafts (24) verschiebbar ist, der durch das Steuermittel (8, 10) des Griffes (6) betätigbar ist, und andererseits fest mit einer längs verlaufenden, geschlitzten, hohlen Auswerferkanone (50) fest verbunden ist, die vom distalen Ende des Gleitmagazins (22) vorspringt und von dem länglichen Element (C) vorspringen kann,
- in seinem rohrförmigen Körper (20) einerseits Mittel (23, 41) zum Spenden von gespeicherten Klammern (A) eine nach dem anderen, wobei das Spenden den Übertritt des Verankerungsstabes (2) der ersten Klammer in eine Aufnahme (55), die in der Verlängerung der Auswerferkanone (50) angeordnet ist, umfasst, und andererseits einen Auswerferstößel (42), der mittels des Steuermittels (8, 10) des Griffs (6) verschiebbar ist und in der Flucht der Auswerferkanone (50) angeordnet ist, um in diese den Verankerungsstab (2) der ersten Klammer zu stoßen,
**dadurch gekennzeichnet, dass:**
- der distale Teil (2a) des Verankerungsstabes (2) der Klammern (A) eine konische Form aufweist und das distale Ende (2c) dieses distalen Abschnitts (2a) eine halbkugelförmige Form aufweist,
- die Oberfläche des distalen Endes der Auswerferkanone (50) entlang einer Ebene angeordnet ist, die im Wesentlichen senkrecht zur Längsachse des rohrförmigen Körpers (20) angeordnet ist.

2. Anordnung nach Anspruch 1, **dadurch gekennzeichnet, dass** die Auswerferkanone (50) aus Kunststoff ist und einstückig mit dem Gleitmagazin (22) geformt ist.

3. Anordnung nach Anspruch 1 oder 2, **dadurch gekennzeichnet, dass** die Klammer (A) aus einem biokompatiblen Kunststoff ist.

4. Anordnung nach dem vorhergehenden Anspruch, **dadurch gekennzeichnet, dass** die Klammer (A) aus einem bioresorbierbaren Kunststoff ist.

5. Anordnung nach dem vorhergehenden Anspruch, **dadurch gekennzeichnet, dass** der bioresorbierbare Kunststoff ein Milchsäurepolymer ist.

6. Anordnung nach einem der vorhergehenden Ansprüche, **dadurch gekennzeichnet, dass** die Länge des vorspringenden Teils der Auswerferkanone (50) im Moment des Auswurfes kleiner oder gleich 3 mm beträgt.

7. Anordnung nach einem der vorhergehenden Ansprüche, **dadurch gekennzeichnet, dass** die gesamte Länge L' der Klammer (A) in ihrer Funktionsstellung kleiner oder gleich 10,5 mm ist.

8. Anordnung nach einem der vorhergehenden Ansprüche, **dadurch gekennzeichnet, dass** die gesamte Höhe H der Klammer (A) in ihrer Funktionsstellung kleiner oder gleich 6,1 mm ist.

9. Anordnung nach einem der vorhergehenden Ansprüche, **dadurch gekennzeichnet, dass** der Griff (4) mit einem doppelten Rücklaufsperrsystem (12, 13, 14, 19a, 19b, 19c) versehen ist, das einen adäquaten Gebrauch des Apparats gewährleistet und eine Ladehemmung vermeidet.

10. Anordnung nach einem der vorhergehenden Ansprüche, **dadurch gekennzeichnet, dass** das längliche Element C ein Schrittsystem (23) aufweist, das dazu ausgelegt ist, die gespeicherten Klammern A um einen Schritt P einer Weite, die gleich der Länge L des Festlegestabs (3) einer Klammer A ist, zu verschieben, wobei das Schrittsystem (23) eine Zahnstange (28) aufweist, die für jeden Auswurf eine Schubbewegung bewirkt und mit einem Schiffchen (32) versehen ist, das an der Zahnstange (28) gleitend angebracht ist, wobei das Schiffchen (32) mit festen Kerben (35) des Gleitmagazins (22) zusammenwirkt, um einen Schritt P auf der Zahnstange (28) für jeden Auswurf vorzurücken.
